# EUROPEAN PATENT SPECIFICATION

(11) **EP 4 213 900 B1**
(45) Date of publication and mention of the grant of the patent: **25.06.2025**
(21) Application number: 21778205.1
(22) Date of filing: 13.09.2021
(51) Int. Cl.: A61L 27/44, A61L 27/50, A61L 31/12, A61L 31/14, A61B 17/80

(54) **DEVICES COMPRISING COMPOSITE MATERIALS**
VORRICHTUNGEN MIT VERBUNDSTOFFMATERIALIEN
DISPOSITIFS COMPRENANT DES MATÉRIAUX COMPOSITES

(30) Priority: 16.09.2020 GB 202014555
(43) Date of publication of application: 26.07.2023
(73) Proprietor: Invibio Device Component Manufacturing Limited, Thornton Cleveleys, Lancashire FY5 4QD (GB)
(72) Inventor: BONHAM, Stephen, Lancashire FY5 4QD (GB)
(74) Representative: Thompson, Nicola Ruth
(86) International application number: PCT/GB2021/052373
(87) International publication number: WO 2022/058719

(56) References cited:
- WO-A1-2014/072983
- WO-A1-2017/029476
- WO-A1-85/04323
- GB-A- 2 405 342

## Description

### BACKGROUND

The present invention relates to a device comprising a compression moulded body portion comprising layers of composite material. The present invention also relates to a method of manufacturing such a device.

Composite materials comprising polymer and reinforcement fibres are known. An example of such a composite material comprises carbon fibre and polyaryletherketone (PAEK). For instance, the composite material comprises carbon fibre and polyetheretherketone (PEEK)

Such composite materials may be used in a range of applications, including, for example, in the manufacture of medical devices. For example, such composite materials may be used to make implantable devices, such as orthopaedic implants.

GB 2 405 342 describes a carbon fibre reinforced bone fixation plate from PEEK, having a fixation zone with hole therethrough for receiving a screw or nail, whereby this zone is formed from unreinforced PEEK.

### BRIEF DESCRIPTION OF THE DRAWINGS

Embodiments of the present invention are described, by way of example, with reference to the following figures, in which:
Figure 1 is a schematic drawing of a lay-up arrangement that may be used in the manufacture of a device by compression moulding;
Figure 2 is a schematic drawing of a fracture plate template used to form a fracture plate that was tested in the Reference Example;
Figure 3 is a schematic drawing of the fracture plate that was tested in the Reference Example;
Figure 4 is a schematic drawing of a fracture plate template used to form a fracture plate that was tested in Example 1.

### DESCRIPTION

According to one aspect of the present invention, there is provided a device comprising a compression moulded body portion comprising layers of composite material comprising reinforcement fibre and polyaryletherketone; wherein at least one layer of composite material has a first region and a second region, wherein the polyaryletherketone content in the first region is higher than the polyaryletherketone content of the second region; wherein the reinforcement fibres of the second region form continuous filaments that extend in substantially the same direction, and wherein these continuous filaments are interrupted in the first region to provide the first region with the higher PAEK content.

The reinforcement fibres of the second region form continuous filaments, i.e. the length of the filaments is substantially uninterrupted throughout the second region of the at least one layer. These continuous filaments extend in substantially the same direction. The continuous filaments are interrupted or discontinued in the first region to provide the first region with an enhanced polyaryletherketone content. Thus, the first region may have a low reinforcement fibre content, or may be substantially free of reinforcement fibre.

The compression moulded body portion comprises a plurality of layers. Preferably, the compression moulded body portion may comprise layers of composite material comprising reinforcement fibre that form continuous filaments. The continuous filaments may extend along a plane (e.g. length or width) of the layer in a substantially uninterrupted manner. The continuous filaments may extend in substantially the same direction in each layer. In some examples, the continuous filaments may extend along at least 80%, preferably at least 90%, more preferably at least 95%, even more preferably at least 98% of a plane (e.g. length or width) of the layer. In some examples, the continuous filaments in one layer are positioned at an angle to the continuous filaments in an adjacent layer.

In an example, the compression moulded body portion may comprise a core portion that comprises at least one layer, for example, layers of composite material comprising reinforcement fibre that form continuous filaments.

The continuous filaments may be interrupted in an outer layer of the compression moulded body to provide the outer layer with a first region having a higher polyaryletherketone content. The continuous filaments in the outer layer may be positioned substantially at right angles to the continuous filaments of the underlying layer. The outer layer may present e.g. an external surface through which, for example, fixation means (e.g. screws) may be driven through the body portion of the device.

According to a further aspect, there is provided a method of manufacturing a device. The method comprises removing a portion of a first layer of a composite material comprising reinforcement fibre and polyaryletherketone to form a first cut-out region in the first layer; contacting a second layer of the composite material with the first layer of composite material, such that at least part of the second layer overlaps with at least part of the first cut-out region; and compression moulding layers of the composite material together, such that some of the polyaryletherketone of the composite material of the second layer flows into the first cut-out region.

The device of the present disclosure comprises a compression moulded body portion comprising layers of composite material. The composite material comprises polyaryletherketone and reinforcement fibre (e.g. carbon fibre). At least one layer of the moulded body portion has a first region having a relatively low ratio of reinforcement fibre to polyaryletherketone. This first region may be formed, for example, during the compression moulding process by cutting out part of the composite material in the layer to form a cut-out region. By cutting out part of the composite material in this way, the continuity of the reinforcement fibres may be interrupted. During the compression moulding, the polyaryletherketone from an adjacent layer flows into the cut-out region, forming a region with an enhanced polyaryletherketone content.

The mechanical properties of the first region of the layer may be different from the mechanical properties of the remainder of the layer (e.g. the second region). For example, the higher polyaryletherketone content in the first region may provide the first region with enhanced flexibility and/or ductility. This may be useful for reducing the risk of crack propagation and/or delamination, for example, when the moulded body portion is subject to insertion torques. Such insertion torques may be applied when screws are driven into apertures (e.g. threaded (e.g. screw holes) or unthreaded holes) in the moulded body portion. Such insertion torques may otherwise cause delamination (e.g. of an outer layer of the compression moulded body portion) and/or cracking, for example, adjacent the aperture. Additionally, any interruption or discontinuity of the continuous fibres may disrupt the stresses travelling along the fibres and between adjacent layers, This may mitigate the risks of delamination and/or crack propagation.

In some instances, the compression moulded body portion may comprise an aperture. A plurality of apertures may be present. The aperture may be configured to receive a fixation device, for example, to secure an object to the compression moulded body or vice-versa. The aperture may be a threaded aperture, for instance, a screw hole. The compression moulded body portion comprises at least two apertures (e.g. screw holes).

In some cases, the first region of the layer of composite material may be adjacent to the aperture. By positioning the first region adjacent the aperture, the risk of crack formation, crack propagation and/or delamination may be reduced because of the increased flexibility provided by the first region. Preferably, the first region may define at least part of the aperture wall. The first region may extend from the aperture to an outer edge of the compression moulded body portion. Such a region may reduce the risk of crack formation and/or crack propagation from the aperture to an outer edge of the compression moulded body portion. In some instances, the compression moulded body portion comprises at least two apertures. The first region may extend between the two apertures. This can reduce the risk of crack formation and crack propagation between apertures, for instance, when the compression moulded body portion is subjected to insertion torques.

In some cases, the layer of composite material that comprises the first region and a second region forms an outer layer of the compression moulded body portion. The outer layer may form an upper and/or a lower surface of the compression moulded body portion. As mentioned above, the first region has an increased polyaryletherketone content. This may reduce the risk of e.g. delamination of outer layer(s) of the compression moulded body portion. In some cases, a plurality of the outer layers of the compression moulded body each comprises a first region having the increased polyaryletherketone content. The first regions of each of these outer layers may overlap to provide a region of increased polyaryletherketone content e.g. that is a plurality of layers thick.

The first region may be substantially free from reinforcement fibre. Where the first region is not substantially free from reinforcement fibre, the volume ratio of reinforcement fibre to polyaryletherketone may be less than 1.3 : 1, preferably less than 0.5 : 1, more preferably less than 0.2 : 1 or less than 0.1 : 1.

The volume ratio of reinforcement fibre to polarylether ketone in the second region (or remainder of the layer) may be 0.5 - 2 : 1. Preferably, the volume ratio of reinforcement fibre to polaryletherketone in the second region (or remainder of the layer) may be 0.8 - 1.5 :1, more preferably 0.9 - 1.2 : 1.

The compression moulded body portion may also comprise at least one layer that is formed from a composite material having substantially the same reinforcement to polyaryletherketone volume ratio throughout. Such a layer preferably does not have a first region having an enhanced polyaryletherketone content. In some cases, the compression moulded body portion may comprise such a layer as a core or middle layer of the compression moulded body portion. The compression moulded body portion may comprise a plurality of such layers as a core or middle layer of the compression moulded body portion. In some cases, the reinforcement to polyaryletherketone volume ratio of such layer(s) is the same as the reinforcement to polyaryletherketone volume ratio of the second region. The volume ratio of reinforcement fibre to polarylether ketone in the such a layer (s) may be 0.5 - 2 : 1. Preferably, the volume ratio of reinforcement fibre to polaryletherketone may be 0.8 - 1.5 :1, more preferably 0.9 - 1.2 : 1.

Preferably, the reinforcement fibre is carbon fibre.

Preferably, the polyaryletherketone is polyetheretherketone (PEEK).

The composite material may be provided in the form of a tape. Layers (also referred to as "plies") may be formed of joined portions of tape. The composite material may take the form of a net or other precursor that can be used to form a compression moulded body portion of a desired shape and/or configuration.

The device may be a medical device, for example, an implantable device. Examples of implantable devices include orthopaedic implants, for instance, fracture plates, trauma plates, spinal implants and other load bearing implantable devices.

The composite material may additionally comprise additives in the matrix An example of additive includes bioactive agents, such as hydroxyapatite, or an image contrast agent, such as barium sulphate. In some examples, the composite material may comprise an image contrast agent. The image contrast agent may be present in all the layers or in selected layers of the composite material. The contrast agent may be an X-ray detectable material. For example, the contrast agent may be barium sulphate.

### Polyaryletherketone

Any suitable polyaryletherketone may be used in the composite material of the present invention.

Suitable polyaryl ether ketone may have repeating units of formula (I) below: 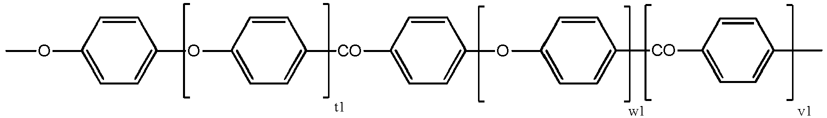 where t1 and w1 are independently represent 0 or 1 and v1 represents 0, 1 or 2.

The polyaryletherketone suitably includes at least 90, 95 or 99 mol % of repeat unit of formula I.

The polyaryletherketone may comprise or consist essentially of a repeat unit of formula I. Preferred polymeric materials comprise (or consist essentially of) a said repeat unit wherein t1=1 , v1=0 and w1=0; t1=0, v1=0 and w1=0; t1=0, w1 =1 , v1=2; or t1=0, v1=1 and w1=0. More preferably, the polyaryletherketone comprises (e.g. consists essentially of) the repeat unit I, wherein t1 =1 , v1 =0 and w1 =0; or t1=0, v1=0 and w1 =0. The most preferred polyaryletherketone comprises (especially consists essentially of) a said repeat unit wherein t1=1 , v1=0 and w1=0.

In preferred embodiments, the polyaryletherketone is selected from polyetheretherketone, polyetherketone, polyetherketoneetherketoneketone and polyetherketoneketone.

In a more preferred embodiment, the polyaryletherketone is polyetheretherketone or PEEK.

In some examples, the polyaryletherketone (e.g. PEEK) may have a Notched Izod Impact Strength (specimen 80mm x 10mm x 4mm with a cut 0.25mm notch (Type A), tested at 23°C, in accordance with ISO180) of at least 4 KJmT⁻², preferably at least 5 KJmT⁻², more preferably at least 6 KJmT⁻² . The Notched Izod Impact Strength, measured as described above, may be less than 10 KJmT⁻², suitably less than 8 KJmT⁻². The Notched Izod Impact Strength, measured as described above, may be at least 3 KJmT⁻², suitably at least 4 KJmT⁻², preferably at least 5 KJmT⁻². The Notched Izod Impact Strength may be less than 50 KJmT⁻², suitably less than 30 KJmT⁻².

The polyaryletherketone (e.g. PEEK) suitably has a melt viscosity (MV) of at least 0.06 kNsm⁻², preferably has a MV of at least 0.09 kNsm⁻², more preferably at least 0.12 kNsm⁻². The polyaryletherketone (e.g. PEEK) may have a MV of less than 1.00 kNsm⁻², preferably less than 0.5 kNsm⁻².

The polyaryletherketone (e.g. PEEK) may have a MV in the range 0.09 to 0.5 kNsm⁻², preferably in the range 0.1 to 0.3 kNsm⁻², preferably having a MV in the range 0.1 to 0.2 kNsm⁻². An MV of 0.15 kNsm⁻² has been found to be particularly advantageous. MV is suitably measured using capillary rheometry operating at 400°C at a shear rate of 1000s⁻¹ using a tungsten carbide die, 0.5mm x 3.175 mm.

In a preferred embodiment, the polyaryletherketone (e.g. PEEK) has a melt viscosity (MV) of 0.09 kNsm⁻² to 0.5 kNsm⁻².

The polyaryletherketone (e.g. PEEK) may be amorphous or semi-crystalline. The polyaryletherketone is preferably crystallizable. The polyaryletherketone is preferably semi-crystalline. The level and extent of crystallinity in a polymer is preferably measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calorimetry (DSC).

The level of crystallinity of said polyaryletherketone (e.g. PEEK) may be at least 1 %, suitably at least 3%, preferably at least 5% and more preferably at least 10%. In especially preferred embodiments, the crystallinity may be greater than 25%. It may be less than 50% or less than 40%.

The main peak of the melting endotherm (Tm) of the polyaryletherketone (if crystalline) may be at least 300°C. Where e.g. PEEK is used, the main peak of the melting endotherm (Tm) may be at least 300°C.

The composite material may comprise any suitable amount of the polyaryletherketone (e.g. PEEK). For example, the composite material may comprise at least 20 volume %, preferably at least 25 volume %, more preferably at least 30 volume %, yet more preferably at least 35 volume %, even more preferably at least 37 volume % and most preferably at least 39 volume % polyaryletherketone (e.g. PEEK). The composite material comprises up to 48 volume % polyaryletherketone (e.g. PEEK). In some embodiments, the composite material may comprise up to 45 volume %, up to 43 volume % polyaryletherketone (e.g. PEEK).

In some embodiments, the composite material may comprise 20 to 48 volume %, preferably 30 to 48 volume %, more preferably 35 to 48 volume %, yet more preferably 37 to 48 volume % or 38 to 48 volume % polyaryletherketone (e.g. PEEK). More preferably, the composite material may comprise 39 to 48 volume %, even more preferably 39 to 45 volume % polyaryletherketone (e.g. PEEK). In some embodiments, the composite material may comprise 39 to 43 volume % polyaryletherketone (e.g. PEEK).

The volume ratio of reinforcement fibre to polyaryletherketone (e.g. PEEK) is 1.1 : 1 to 1.5 : 1, for example, 1.2: 1 to 1:4 :1.

### Reinforcement fibres

Any suitable reinforcement fibre may be used. The fibres used may be selected from inorganic or organic fibrous materials. The fibres may have a melting or decomposition temperature of greater than 200 °C, for example, greater than 250 °C or greater than 300 °C. In some embodiments, the fibres may have a melting temperature of greater than 350 °C or 500 °C. Examples of suitable fibres include aramid fibres, carbon fibre, glass fibre, carbon fibre, silica fibre, zirconia fibre, silicon nitride fibre, boron fibre and potassium titanate fibre. Most preferred fibres are carbon fibres.

The reinforcement fibre (e.g. carbon fibre) may have a tensile strength of greater than 4200 MPa, preferably greater than 4500 MPa, more preferably greater than 4800 MPa..

The reinforcement fibre (e.g. carbon fibre) may have a tensile modulus of greater than 200 GPa, preferably greater than 230 GPa, more preferably greater than 240 GPa.

The reinforcement fibre (e.g. carbon fibre) may have a strain at failure of greater than 1.1%, preferably, greater than 1.2%, 1.4% or 1.6% The reinforcement fibre (e.g. carbon fibre) may have a strain at failure of less than 2.2%, for instance, less than 2.0% or 1.9%. In some embodiments, reinforcement fibre (e.g. carbon fibre) may have a strain at failure of 1.2 to 2.2%, for example, 1.4 to 2.0 % or 1.6 to 1.9%. In one embodiment, the reinforcement fibre (e.g. carbon fibre) may have a strain at failure of 1.7 to 1.9%.

The reinforcement fibre (e.g. carbon fibre) may have a mass per unit length of 0.1 to 1.0 g/m, for example, 0.2 to 0.8 g/m. In some embodiments, the 0.2 to 0.5 g/m.

The reinforcement fibre (e.g. carbon fibre) may have a density of greater than 1.65 g/cm³, preferably greater than 1.70 g/cm³. The reinforcement fibre (e.g. carbon fibre) may have a density of less than 1.85 g/cm³, preferably less than 1.80 g/cm³. In some embodiments, the reinforcement fibre (e.g. carbon fibre) may have a density of 1.70 to 1.85 g/cm³, for example, 1.75 to 1.80 g/cm³, or 1.78 to 1.79 g/cm³.

The reinforcement fibre (e.g. carbon fibre) may be provided in the form of a continuous tow. Any suitable tow size may be used. The tow size indicates the number of filaments in the tow. In some embodiments, the tow size may be 1000 to 24,000. In one embodiment, a tow size of 6000 to 12,000 may be employed.

Examples of suitable reinforcement fibre include carbon fibres supplied, for example, by Hexcel^{®} under the trademark HexTow^{®}.

The reinforcement fibre (e.g. carbon fibre) may be present in an amount of 30 to 68 volume %, preferably 40 to 65 volume %. Preferably, the reinforcement fibre may be present in an amount of 50 to 62 volume %, for instance, 52 to 58 volume % based on the total volume of the composite material.

The reinforcement fibre (e.g. carbon fibre) may be formed into filaments. Any suitable method may be employed. For example, the reinforcement fibres may be twisted or braided to form filaments. Where the composite material is formed into tape, the filaments may be substantially aligned along the longitudinal axis of the tape.

The amount of reinforcement fibre (e.g. carbon fibre) in the composite material can be controlled within a narrow range to enable the composite material to provide an optimised balance of mechanical properties.

In some embodiments, the composite material also comprises a contrast agent, e.g. barium sulphate. For example, barium sulphate may be present in the composite material in an amount of 2 to 20 weight % of the total weight of the composite material, for instance, 3 to 10 weight %. In a preferred embodiment, the amount of barium sulphate may be 4 to 8 weight %, more preferably 4 to 6 weight %. In a most preferred embodiment, the amount of barium sulphate may be 5 weight %.

By using controlled amounts of the reinforcement fibre (e.g. carbon fibre) in combination with contrast agent, e.g. barium sulphate, it may also be possible to vary the properties of the composite material in terms of its imageability under e.g. X-ray. For example, while barium sulphate may provide sufficient radio-opacity for an implantable device to be detected under e.g. X-ray, the amount of reinforcement fibre (e.g. carbon fibre) is controlled within narrow limits to provide or maintain sufficient translucency to allow the fracture in the underlying bone to be detected under imaging techniques e.g. X-ray.

Moreover, by using controlled amounts of the reinforcement fibre (e.g. carbon fibre) in combination with barium sulphate, the radio-translucency of the composite material may be optimized to reduce interference, such that dosing accuracy during radiotherapy can be maintained.

### Contrast Agent

Any suitable contrast agent may be employed. Preferably, the contrast agent is detectable by X-ray. In some embodiments, the contrast agent comprises barium. For instance, the contrast agent may be barium sulphate.

Barium sulphate is a contrast medium that allows the composite material to be detected under imaging techniques, for example, X-ray. Accordingly, when the composite material is used in the manufacture of an implantable device, the device may be detected under e.g. X-ray.

The barium sulphate may have a D₁₀ particle size in the range of 0.1 to 1.0 microns; a D₅₀ particle size in the range of 0.5 to 2.0 microns and a D₉₀ particle size in the range of 1.0 to 5 microns. The D₁₀ particle size may be in the range of 0.1 to 0.6 microns, preferably 0.2 to 5 microns. The D₅₀ particle size may be in the range of 0.7 to 1.5 microns, preferably 0.8 to 1.3 microns. The D₉₀ particle size may be in the range of 1.5 to 3 microns, preferably in the range of 2.0 to 2.5 microns.

Suitable X-ray grade barium sulphate may be available from Merck-Millipore^{®}.

Any suitable amount of contrast agent e.g. barium sulphate may be used. For example, contrast agent e.g. barium sulphate may be present in the composite material in an amount of 2 to 20 weight %, preferably, 3 to 15 weight %, for instance, 3 to 10 weight %. In a preferred embodiment, the amount of contrast agent e.g. barium sulphate may be 3 to 8 weight %, more preferably 3 to 5 or 4 to 6 weight %. In a most preferred embodiment, the amount of contrast agent e.g. barium sulphate may be 5 weight %.

The amount of contrast agent e.g. barium sulphate may be controlled, such that the radio-translucency of the composite material is optimized to reduce interference. This can allow dosing accuracy during radiotherapy to be maintained.

Furthermore, by controlling the relative amounts of the reinforcement fibre (e.g. carbon fibre) to barium sulphate, it may also be possible to vary the properties of the composite material in terms of its imageability under e.g. X-ray. For example, the relative amounts of the reinforcement fibre (e.g. carbon fibre) to barium sulphate may be controlled to allow the implantable device to be detected under e.g. X-ray, while maintaining sufficient translucency to allow the fracture in the underlying bone to be detected.

Moreover, the relative amounts of the reinforcement fibre (e.g. carbon fibre) to barium sulphate may be controlled, such that the radio-translucency of the composite material is optimized to reduce interference. This can allow dosing accuracy during radiotherapy to be maintained.

### Composite Material

The composite material may be formed as tape. For example, the reinforcement fibre (e.g. carbon fibre) may be combined with the polyaryletherketone (e.g. PEEK) and formed into a tape. A plurality of tapes may be joined to form a layer and the layers may be compression moulded to form the compression moulded body portion of the device. In an embodiment, the polyaryletherketone (e.g. PEEK) may be heated to above its softening or melting temperature to melt or soften the polymer around the fibres to form the composite. The molten or soften polymer is then compressed around the fibres.

When heat is applied, suitable temperatures include temperatures of 320°C and above, preferably, of 330°C and above, more preferably, of 340°C and above. In some embodiments, compression moulding may be carried out at temperatures of 320 to 450°C, preferably 330 to 400°C, more preferably 340 to 380 °C and yet more preferably 350 to 370°C. Suitably, pressures of at least 1.5 MPa or at least 2 MPa may be applied. Examples of suitable pressures range from 1.5 to 10 MPa, for instance, 2 to 8 MPa.

The tape or layer formed using the composite material of the present invention may have a thickness of 10 microns to 1 mm, preferably 100 to 300 microns, more preferably 140 to 200 microns.

### Device

As mentioned above, an aspect of the present invention is a method of manufacturing a device. The method comprises removing a portion of a first layer of a composite material comprising reinforcement fibre and polyaryletherketone to form a first cut-out region in the first layer; contacting a second layer of the composite material with the first layer of composite material, such that at least part of the second layer overlaps with at least part of the first cut-out region; and compression moulding layers of the composite material together, such that some of the polyaryletherketone of the composite material of the second layer flows into the first cut-out region.

When some of the polyaryletherketone of the composite material in of the second layer flows into the first cut-out region, the polyaryletherketone in the first cut-out region is enhanced. This polyaryletherketone-rich region may form the first region of the first layer. The remainder of the first layer may form the second region of the first layer.

As discussed above, the polyaryletherketone-rich region may have enhanced flexibility and/or ductility. This may be useful for reducing the risk of crack propagation and/or delamination, for example, when the moulded body portion is subject to insertion torques that may otherwise cause delamination (e.g. of an outer layer of the compression moulded body portion) and/or cracking. Accordingly, in some embodiments of the method of the present disclosure, portions of a first layer of composite material may be cut away to form cut-out region(s) that correspond to regions of the compression moulded body portion of the device that are susceptible to cracking or other damage e.g. when the device is subjected to insertion torques. During compression moulding, the polyaryletherketone from an adjacent layer (e.g. second layer) of composite material is compression moulded can flow into the cut-out region(s) to form polyaryletherketone-rich regions in the first layer. As noted above, these polyaryletherketone-rich regions provide enhanced flexibility and/or ductility to reduce the risk of damage in these regions.

The regions of the compression moulded body portion of the device that are susceptible to cracking or other damage (e.g. when the device is subjected to insertion torques) may be regions adjacent to or surrounding an aperture (e.g. screw hole). Accordingly, in some embodiments, portions of a first layer of composite material may be cut away to form cut-out region(s) that correspond to regions adjacent to or that at least partially surround an aperture(s) (e.g. screw hole). After compression moulding, the resulting first region(s) will be located adjacent to the aperture(s) or may at least partially define at least part of the aperture wall. This may reduce the risk of cracking or other damage in the vicinity of the aperture(s).

In some embodiments, portions of a first layer of composite material may be cut away to form cut-out region(s) that correspond to regions that extend from the aperture(s) (e.g. screw hole) to an edge of the compression moulded body portion. Accordingly, the resulting first region may extend from the aperture to an outer edge of the compression moulded body portion. Such a region may reduce the risk of crack formation and/or crack propagation from the aperture to an outer edge of the compression moulded body portion.

In embodiments where the compression moulded body comprises two or more apertures (e.g. screw holes), portions of a first layer of composite material may be cut away to form cut-out region(s) that correspond to regions that connect the two or more apertures (e.g. screw holes). After compression moulding, the resulting first region(s) will be located in regions that extend between the two or more apertures (e.g. screw holes). This can help to prevent cracks from propagating between (e.g. adjacent or nearby) apertures in the compression moulded body portion.

The first layer of composite material that is cut to form cut-out regions may be used to form an outer layer of the compression moulded body portion. The outer layer may form an upper and/or a lower surface of the compression moulded body portion. It may be desirable to have first regions present on an outside surface of the compression moulded body portion, as this may reduce the risk of delamination of the outer layer(s) of the device. Such outer layer(s) may otherwise have an increased risk of delamination when the compression moulded body portion is subjected to insertion torques.

In some cases, the composite material may be cut out from a stack of layers (i.e. overlying layers) of composite material. During compression moulding, polyaryletherketone from adjacent layers of composite material may flow to fill the cut-out region. This forms polyaryletherketone-rich region that is a plurality of layers thick in the compression moulded body portion. This polyaryletherketone-rich region may be 2 to 10 layers thick, preferably 2 to 5 layers thick, more preferably 1 to 2 layers thick. The number of layers may make up to 50% of the thickness of the plate, more preferable less than 30% of the thickness, more preferably less than 10% of the plate thickness but at least 1 ply layer. This polyaryletherketone-rich region may form part of the outer surface of the compression moulded body portion. By confining this polyaryletherketone-rich region to, for example, only a proportion (e.g. outer layer) of the compression moulded body, other functional properties of the body can be varied/controlled by varying or controlling the material properties of the remaining layers of the body.

As mentioned above, the layers of composite material may be formed from tape of the composite material. Where tape is employed to form a layer, multiple tapes may be aligned unidirectionally in the layer. Multiple layers of tape may then be overlaid in a lay-up arrangement, and compression moulded to form a laminate, for example, in the shape of the body of the device. Where the reinforcement fibres form filaments, the filaments may be aligned along the length of the tape. The filaments may be continuous and hence uninterrupted along the length of the tape.

In some embodiments, the tape in a first layer may be aligned unidirectionally along an axis. Tape in a second layer may be aligned unidirectionally at an angle to the axis of the first layer. In the third layer of the lay-up, the tape may be unidirectionally aligned at an angle that is different from the angle of the second layer and/or the first layer. These and subsequent layers may be oriented at angles depending on the desired properties and/or shape of the device. The lay-up arrangement may be determined using computer software, depending on the shape, configuration and/or properties of the final device. The outer layer of the lay-up arrangement may be angled relative to the preceding layer in a manner to reduce the risk of crack propagation and/or delamination.

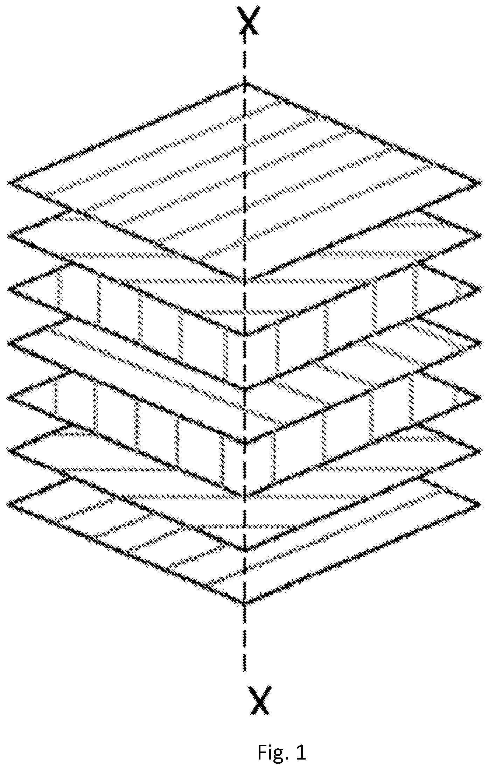

In one example, the tape in the first layer are aligned at 0° to the axis. The tape in the second layer may be aligned at 90° to the axis, while the third and fourth layers may be aligned at 0° and 90° to the axis, respectively. This alternating pattern may be continued throughout the laminate. In an alternative embodiment, the tape in the first layer are aligned at 0° to the axis. The tape in the second layer may be aligned at 45° to the axis, while the third layer may be aligned at 90° to the axis. The fourth layer may be aligned at -45 ° to the axis, while the fifth layer may be aligned at 0° to the axis and so on. Figure 1 illustrates an example of a suitable lay-up for producing a laminate for the device. In some examples, the orientation of the tapes in the outer layer may be aligned at an angle to tapes in the preceding layer, for example, at right angles to the preceding layer. This may help to reduce the risk of cracking and/or delamination.

The tape in the lay-up arrangement may be compression moulded to form the device or part thereof. Compression moulding may be carried out by applying heat and pressure. The resulting part may then be cooled, for example, under pressure.

When heat is applied, suitable temperatures include temperatures of 320°C and above, preferably, of 330°C and above, more preferably, of 340°C and above. In some embodiments, compression moulding may be carried out at temperatures of 320 to 450°C, preferably 330 to 400°C, more preferably 340 to 380 °C and yet more preferably 350 to 370°C. Suitably, pressures of at least 1.5 MPa or at least 2 MPa may be applied. Examples of suitable pressures range from 1.5 to 10 MPa, for instance, 2 to 8 MPa.

In some embodiments, after heat and pressure are applied, the part is rapidly cooled, preferably to between 140°C and 200°C. In some embodiments, cooling may be carried out under pressure.

In some examples, the layers comprise the same polyaryletherketone (e.g. PEEK). The layers may be formed of the same composition save for the first region(s) of the compression moulded body portion, which have a richer polyaryletherketone content.

Some of the layers of the device may be free from barium sulphate, while some of the layers may comprise barium sulphate.

In a preferred embodiment, at least an outer region (e.g. outer or external surface) of the device comprises barium sulphate. This can allow the outer region of the device to be detectable using imaging techniques, for example, X-ray. In some instances, an inner region of the device may comprise barium sulphate. In some instances, barium sulphate may be present throughout the device.

Where the device has a body having a variable cross-section, the device may be formed using the method described in WO 2017/029476. For example, the body may be formed of a first external layer and a second external layer. Packing layers may be positioned between the first external layer and second external layer, and an insert layer(s) may be positioned within the packing layers to provide increased depth to a portion of the body. The layers may be compression moulded together to form the body of the device. The first external layer may contact the second external layer, so as to reduce exposure of the ends of the packing layers and insert layer(s), thereby reducing the risk of delamination of the moulded part.

The device may be a medical device. Preferably, the device may be an implantable device. Examples include orthopaedic implants, for instance, fracture plates and/or trauma plates, intramedullary nails, spinal implants such as cages, rods and screws, and other load bearing or bone contacting implants. In a preferred embodiment, the device is a fracture plate or trauma plate.

The device may include one or more apertures. For example, the device may include a threaded aperture e.g. for receiving a fixation screw for securing the device to an underlying structure. In the case of an orthopaedic implant, the device may include a threaded aperture for receiving a screw for securing the device to underlying bone. In one embodiment, the device is an implantable implant comprising one or more threaded apertures. In a preferred embodiment, the device may be a fracture plate or trauma plate comprising one or more threaded apertures. The implant may be resistant to damage on application of relatively high insertion torques.

These and other aspects of the present invention will now be described with reference to the accompanying drawings.

Referring to Figure 1, this is a schematic drawing of an example lay-up arrangement of layers of tape formed from the composite material of an embodiment of the present invention. Starting from the bottom of the lay-up as shown, the first layer is formed of tape that is unidirectionally aligned along an axis (0°). The second layer is formed of tape that is unidirectionally aligned at 45° to the axis of the first layer. The third layer is formed of tape 14c that is unidirectionally aligned at -45° to the axis of the first layer. The fourth layer is formed of tape 14d that is unidirectionally aligned at 90° to the axis of the first layer. The pattern is repeated so that the overall structure has the following alignment: 0°, 45°, -45°, 90°, -45°, 45° and 0. The resulting laminate may be compression moulded under heat and pressure to form the compression moulded body portion of a device, for example, a fracture plate

Although not shown, a portion of the bottom and/or final layer may be removed to form cut-out regions corresponding to regions surrounding the threaded apertures. When the laminate is compression moulded, polyaryletherketone from adjacent layers flows into the cut-out regions, forming polyaryletherketone-rich regions surrounding the threaded apertures. These polyaryletherketone-rich regions provide increased flexibility and/or ductility, reducing the risk of cracking and/or delamination in the outer layer of the resulting device.

### Examples

### Reference Example

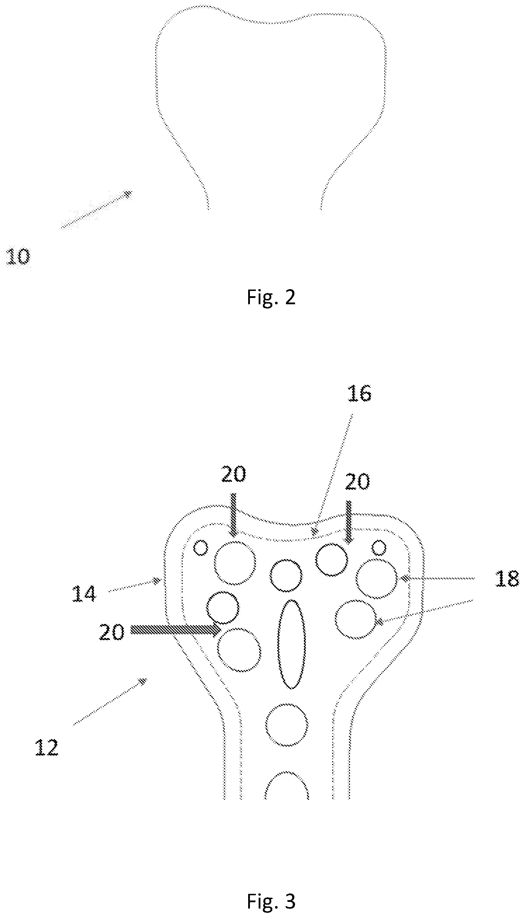

A fracture plate template 10 shown schematically in Figure 2 was formed by compression moulding layers of a composite material comprising PEEK and carbon fibre.

Figure 3 is a schematic drawing showing of how the fracture plate template 10 shown in Figure 2 was used to form a fracture plate 12. The outer border 14 in Figure 3 corresponds to the border of the fracture plate template 10 of Figure 2. To form the fracture plate 12, the fracture plate template 10 of Figure 2 was cut down to the size shown by the dotted boundary 16 in Figure 3. Screw holes 18 were also made in the fracture plate 12.

A screw (not shown) was inserted into the screw holes 18 and an insertion torque applied to the plate 12. The plate12 began to crack in regions adjacent the screw holes 18. An insertion torque of 1.36 Nm was reached before the holes failed functionally (i.e. the screw was no longer secured and the torque required to turn the screw started to decrease).. The arrows 20 in Figure 3 illustrate the regions of the plate 12 that were most prone to cracking when screws were inserted into the holes 18.

### Example

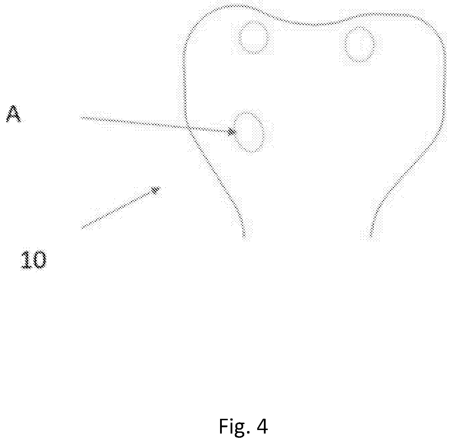

The above procedure of the Reference Example was repeated except that, before compression moulding the fracture plate template 10, a portion of the outermost layer of composite material was cut out in regions "A" of Figure 4. When the layers were compression moulded, some of the PEEK from adjacent layers flowed into the cut-out to form a PEEK-rich region extending between threaded apertures in region "A" shown in Figure 4. The position of regions "A" may approximately correspond to the locations 20 found in the Reference Example to be more prone to failure.

A screw (not shown) was inserted into each of the screw holes 18 and an insertion torque applied. No cracking or delamination was observed at functional failure of the holes 18. The holes 18 were also able to withstand higher insertion torque values before failure, with hole functionally failing at a torque of 1.8 Nm.

### Definitions

It is noted that, as used in this specification and the appended claims, the singular forms "a," "an," and "the" include plural referents unless the content clearly dictates otherwise.

As used herein, the term "about" is used to provide flexibility to a range endpoint by providing that a given value may be "a little above" or "a little below" the endpoint. The degree of flexibility of this term can be dictated by the particular variable and can be determined based on experience and the associated description herein.

As used herein, a plurality of items, structural elements, compositional elements, and/or materials may be presented in a common list for convenience. However, these lists should be construed as though each individual member of the list is individually identified as a separate and unique member. Thus, no individual member of such list should be construed as a *de facto* equivalent of any other member of the same list solely based on their presentation in a common group without indications to the contrary.

Concentrations, dimensions, amounts, and other numerical data may be presented herein in a range format. It is to be understood that such range format is used merely for convenience and brevity and should be interpreted flexibly to include the numerical values explicitly recited as the limits of the range, and also to include all the individual numerical values or sub-ranges encompassed within that range as if the numerical value and sub-range is recited. For example, a weight ratio range of about 1 wt% to about 20 wt% should be interpreted to include the explicitly recited limits of 1 wt% and about 20 wt%, and also to include individual weights such as 2 wt%, 11 wt%, **14** wt%, and sub-ranges such as 10 wt% to 20 wt%, 5 wt% to 15 wt%, etc.

## Claims

1. A device comprising a compression moulded body portion comprising layers of composite material comprising reinforcement fibre and polyaryletherketone; wherein at least one layer of composite material has a first region and a second region, wherein the polyaryletherketone (PAEK) content in the first region is higher than the polyaryletherketone content of the second region; wherein the reinforcement fibres of the second region form continuous filaments that extend in substantially the same direction, and wherein these continuous filaments are interrupted in the first region to provide the first region with the higher PAEK content.

2. The device as claimed in any one of the preceding claims, wherein the volume ratio of reinforcement fibre to PAEK in the second region is 0.5 - 2 : 1.

3. The device as claimed in any one of the preceding claims, wherein the first region is substantially free from reinforcement fibre.

4. The device as claimed in any one of the preceding claims, wherein the compression moulded body portion comprises an aperture.

5. The device as claimed in claim 4, wherein the aperture is a screw hole.

6. The device as claimed in any one of claims 4 or 5, wherein the first region is adjacent to the aperture.

7. The device as claimed in claim 6, wherein the first region defines at least part of the aperture wall.

8. The device as claimed in claim 6 or 7, wherein the first region extends from the aperture to an outer edge of the compression moulded body portion.

9. The device as claimed in claim 4, which comprises at least two apertures and wherein the first region extends between the two apertures.

10. The device as claimed in any one of the preceding claims, wherein the layer of composite material that has the first region and a second region forms an outer layer of the compression moulded body.

11. The device as claimed in any one of the preceding claims, wherein the polyaryl ether ketone is polyether ether ketone (PEEK) and wherein the reinforcement fibre is carbon fibre.

12. The device as claimed in any one of the preceding claims, which is an implantable medical device.

13. The device as claimed in claim 12, which is a bone plate.

14. The device as claimed in any one of the preceding claims, wherein the compression moulded body portion comprises layers of composite material comprising reinforcement fibre that form continuous filaments, said continuous filaments being interrupted in an outer layer of the compression moulded body to provide the outer layer with a first region having a higher PAEK content.

15. The device as claimed in claim 14, wherein the continuous filaments in one layer are positioned at an angle to the continuous filaments in an adjacent layer.

16. The device as claimed in claim 14 or 15, wherein the continuous filaments in the outer layer are positioned substantially at right angles to the continuous filaments of the underlying layer.

17. A method of manufacturing a device as claimed in any one of the preceding claims, said method comprising: removing a portion of a first layer of a composite material comprising reinforcement fibre and polyaryletherketone to form a first cut-out region in the first layer, contacting a second layer of the composite material with the first layer of composite material, such that at least part of the second layer overlaps with at least part of the first cut-out region, and compression moulding layers of the composite material together, such that some of the polyaryletherketone of the composite material of the second layer flows into the first cut-out region.

## Patentansprüche

1. Vorrichtung, umfassend einen formgepressten Körperabschnitt, umfassend Schichten aus Verbundmaterial, umfassend Verstärkungsfasern und Polyaryletherketon; wobei mindestens eine Schicht aus Verbundmaterial einen ersten Bereich und einen zweiten Bereich aufweist, wobei der Polyaryletherketon-Gehalt (PAEK-Gehalt) in dem ersten Bereich höher als der Polyaryletherketon-Gehalt in dem zweiten Bereich ist; wobei die Verstärkungsfasern des zweiten Bereichs kontinuierliche Filamente ausbilden, die sich im Wesentlichen in die gleiche Richtung erstrecken, und wobei diese kontinuierlichen Filamente in dem ersten Bereich unterbrochen sind, um den ersten Bereich mit dem höheren PAEK-Gehalt zu versehen.

2. Vorrichtung nach einem der vorstehenden Ansprüche, wobei das Volumenverhältnis von Verstärkungsfaser zu PAEK in dem zweiten Bereich 0,5-2 : 1 beträgt.

3. Vorrichtung nach einem der vorstehenden Ansprüche, wobei der erste Bereich im Wesentlichen frei von Verstärkungsfasern ist.

4. Vorrichtung nach einem der vorstehenden Ansprüche, wobei der formgepresste Körperabschnitt eine Öffnung aufweist.

5. Vorrichtung nach Anspruch 4, wobei die Öffnung ein Schraubenloch ist.

6. Vorrichtung nach einem der Ansprüche 4 oder 5, wobei der erste Bereich an die Öffnung angrenzt.

7. Vorrichtung nach Anspruch 6, wobei der erste Bereich mindestens einen Teil der Öffnungswand definiert.

8. Vorrichtung nach Anspruch 6 oder 7, wobei sich der erste Bereich von der Öffnung bis zu einer Außenkante des formgepressten Körperabschnitts erstreckt.

9. Vorrichtung nach Anspruch 4, die mindestens zwei Öffnungen umfasst und wobei sich der erste Bereich zwischen den zwei Öffnungen erstreckt.

10. Vorrichtung nach einem der vorstehenden Ansprüche, wobei die Schicht aus Verbundmaterial, die den ersten Bereich und einen zweiten Bereich aufweist, eine Außenschicht des formgepressten Körpers ausbildet.

11. Vorrichtung nach einem der vorstehenden Ansprüche, wobei das Polyaryletherketon Polyetheretherketon (PEEK) ist und die Verstärkungsfaser Kohlenstofffaser ist.

12. Vorrichtung nach einem der vorstehenden Ansprüche, die eine implantierbare medizinische Vorrichtung ist.

13. Vorrichtung nach Anspruch 12, die eine Knochenplatte ist.

14. Vorrichtung nach einem der vorstehenden Ansprüche, wobei der formgepresste Körperabschnitt Schichten aus Verbundmaterial umfasst, umfassend Verstärkungsfasern, die kontinuierliche Filamente ausbilden, wobei die kontinuierlichen Filamente in einer Außenschicht des formgepressten Körpers unterbrochen sind, um die Außenschicht mit einem ersten Bereich, der einen höheren PAEK-Gehalt aufweist, zu versehen.

15. Vorrichtung nach Anspruch 14, wobei die kontinuierlichen Filamente in einer Schicht in einem Winkel zu den kontinuierlichen Filamenten einer angrenzenden Schicht positioniert sind.

16. Vorrichtung nach Anspruch 14 oder 15, wobei die kontinuierlichen Filamente in der Außenschicht im Wesentlichen in rechten Winkeln zu den kontinuierlichen Filamenten der darunterliegenden Schicht positioniert sind.

17. Verfahren zum Herstellen einer Vorrichtung nach einem der vorstehenden Ansprüche, das Verfahren umfassend: Entfernen eines Abschnitts einer ersten Schicht aus einem Verbundmaterial, umfassend Verstärkungsfasern und Polyaryletherketon, um einen ersten Ausschnittbereich in der ersten Schicht auszubilden, Inkontaktbringen einer zweiten Schicht aus dem Verbundmaterial mit der ersten Schicht aus Verbundmaterial, derart, dass mindestens ein Teil der zweiten Schicht mindestens einen Teil des ersten Ausschnittbereichs überlappt, und Zusammenformpressen von Schichten aus dem Verbundmaterial, derart, dass etwas von dem Polyaryletherketon des Verbundmaterials der zweiten Schicht in den ersten Ausschnittbereich fließt.

## Revendications

1. Dispositif comprenant une portion de corps moulé par compression comprenant des couches de matériau composite comprenant une fibre de renfort et une polyaryléthercétone ; dans lequel au moins une couche de matériau composite a une première région et une seconde région, dans lequel la teneur en polyaryléthercétone (PAEK) dans la première région est supérieure à la teneur en polyaryléthercétone de la seconde région ; dans lequel les fibres de renfort de la seconde région forment des filaments continus qui s'étendent sensiblement dans la même direction, et dans lequel ces filaments continus sont interrompus dans la première région pour fournir à la première région la teneur plus élevée en PAEK.

2. Dispositif selon l'une quelconque des revendications précédentes, dans lequel le rapport volumique de la fibre de renfort au PAEK dans la seconde région va de 0,5 à 2 : 1.

3. Dispositif selon l'une quelconque des revendications précédentes, dans lequel la première région est sensiblement dépourvue de fibre de renfort.

4. Dispositif selon l'une quelconque des revendications précédentes, dans lequel la portion de corps moulé par compression comprend une ouverture.

5. Dispositif selon la revendication 4, dans lequel l'ouverture est un trou de vis.

6. Dispositif selon l'une quelconque des revendications 4 ou 5, dans lequel la première région est adjacente à l'ouverture.

7. Dispositif selon la revendication 6, dans lequel la première région définit au moins une partie de la paroi d'ouverture.

8. Dispositif selon la revendication 6 ou 7, dans lequel la première région s'étend de l'ouverture à un bord externe de la portion de corps moulé par compression.

9. Dispositif selon la revendication 4, qui comprend au moins deux ouvertures et dans lequel la première région s'étend entre les deux ouvertures.

10. Dispositif selon l'une quelconque des revendications précédentes, dans lequel la couche de matériau composite qui a la première région et une seconde région forme une couche externe du corps moulé par compression.

11. Dispositif selon l'une quelconque des revendications précédentes, dans lequel la polyaryléthercétone est une polyéther-éther-cétone (PEEK) et dans lequel la fibre de renfort est de la fibre de carbone.

12. Dispositif selon l'une quelconque des revendications précédentes, qui est un dispositif médical implantable.

13. Dispositif selon la revendication 12, qui est une plaque osseuse.

14. Dispositif selon l'une quelconque des revendications précédentes, dans lequel la portion de corps moulé par compression comprend des couches de matériau composite comprenant une fibre de renfort qui forment des filaments continus, lesdits filaments continus étant interrompus dans une couche externe du corps moulé par compression pour fournir à la couche externe une première région ayant une teneur plus élevée en PAEK.

15. Dispositif selon la revendication 14, dans lequel les filaments continus dans une couche sont positionnés selon un angle par rapport aux filaments continus dans une couche adjacente.

16. Dispositif selon la revendication 14 ou 15, dans lequel les filaments continus dans la couche externe sont positionnés sensiblement selon des angles droits par rapport aux filaments continus de la couche sous-jacente.

17. Procédé de fabrication d'un dispositif selon l'une quelconque des revendications précédentes, ledit procédé comprenant : la retrait d'une partie d'une première couche d'un matériau composite comprenant une fibre de renfort et une polyaryléthercétone pour former une première région de découpe dans la première couche, la mise en contact d'une seconde couche du matériau composite avec la première couche de matériau composite, de telle sorte qu'au moins une partie de la seconde couche chevauche au moins une partie de la première région de découpe, et le moulage par compression des couches du matériau composite ensemble, de telle sorte qu'une partie de la polyaryléthercétone du matériau composite de la seconde couche s'écoule dans la première région de découpe.
